(19) Europäisches Patentamt / European Patent Office / Office européen des brevets

(11) **EP 2 408 366 B1**

(12) **FASCICULE DE BREVET EUROPEEN**

(45) Date de publication et mention de la délivrance du brevet:
**28.11.2018 Bulletin 2018/48**

(21) Numéro de dépôt: **10709236.3**

(22) Date de dépôt: **19.03.2010**

(51) Int Cl.:
*A61B 5/103* (2006.01)    *A61B 5/11* (2006.01)

(86) Numéro de dépôt international:
**PCT/EP2010/053632**

(87) Numéro de publication internationale:
**WO 2010/106175 (23.09.2010 Gazette 2010/38)**

(54) **DISPOSITIF ET PROCEDE DE DETECTION ET DE SUIVI D'UNE LESION DU LIGAMENT CROISE ANTERIEUR**

VORRICHTUNG UND VERFAHREN ZUM NACHWEIS UND ZUR ÜBERWACHUNG EINER LÄSION DES VORDEREN KREUZBANDS

DEVICE AND METHOD FOR DETECTING AND MONITORING A LESION OF THE ANTERIOR CRUCIATE LIGAMENT

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **20.03.2009 FR 0951814**

(43) Date de publication de la demande:
**25.01.2012 Bulletin 2012/04**

(73) Titulaire: **Les Hospices Civils de Lyon**
**69223 Lyon Cedex 02 (FR)**

(72) Inventeurs:
• **Bordet, Bertrand**
**69009 Lyon (FR)**
• **Luciani, Jean-François**
**69001 Lyon (FR)**

(74) Mandataire: **Cabinet Plasseraud**
**235 Cours Lafayette**
**69006 Lyon (FR)**

(56) Documents cités:
**EP-A- 0 568 148     DE-A1- 3 636 843**
**US-A- 4 583 554     US-A- 4 583 555**
**US-A- 5 662 121**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### DOMAINE DE L'INVENTION

La présente invention concerne un dispositif de détection et de suivi d'une lésion, telle qu'une rupture, par exemple, du ligament croisé antérieur dit « LCA », permettant d'effectuer de façon mécanique et à distance un test de Lachman apte à mettre en évidence un tiroir inhabituel, en flexion du genou suivant un angle d'environ 20°, témoignant d'une rupture du ligament croisé antérieur.

### ARRIERE PLAN DE L'INVENTION

Dans le domaine de la traumatologie, il est bien connu d'effectuer un test de Lachman afin de rechercher un tiroir antéro-postérieur inhabituel, en flexion du genou suivant un angle d'environ 20°, témoignant d'une rupture du ligament croisé antérieur. En effet, le tiroir physiologique antéro-postérieur du genou est essentiellement contrôlé par le ligament croisé antérieur de sorte que, en cas d'atteinte du ligament croisé, ce tiroir est modifié.

Ce test de Lachman consiste à empoigner d'une main l'extrémité distale du fémur et, de l'autre main, à empoigner l'extrémité proximale du tibia, puis à faire avancer comparativement le tibia sous le fémur. Ce test s'effectue pour le patient en décubitus dorsal, les muscles de la cuisse étant relâchés, le genou fléchi à 20°, une main fixant la partie inférieure de la cuisse, l'autre main tenant fermement la partie supérieure de la jambe. L'examinateur réalise alors une traction vers l'avant sur le segment jambier tout en immobilisant la cuisse.

Ce test présente l'inconvénient d'être subjectif et d'être difficilement réalisable lors de l'acquisition d'une image médicale par rayons X ou par résonance magnétique (IRM).

Afin de s'affranchir du caractère subjectif du test de Lachman, il a déjà été imaginé des dispositifs permettant la mesure radiologique dynamique du tiroir.

C'est le cas, notamment, du dispositif commercialisé par la société Télos, qui est constitué d'un bâti métallique comportant deux points d'appui fixes pour positionner exactement le membre inférieur, la position étant contrôlable à tout moment par la scopie télévisée, et un poussoir dont la position est réglable en hauteur, permettant d'exercer précisément une force de contrainte contrôlée au Newton près par un cadran à affichage digital.

Le patient est positionné sur une table de radiographie télévisée et le cadre métallique du dispositif Télos est positionné sur la jambe du patient qui est installé en décubitus latéral sur le côté à examiner. Le genou du patient est fléchi à 20° environ, la cuisse bloquée en avant juste au-dessus de la rotule et la cheville est également bloquée en avant, la position étant contrôlée par scopie. La poussée s'effectue précisément à la partie haute de la face postérieure de la jambe, le poussoir apparaissant à la hauteur du rebord tibial postérieur en scopie télévisée. La poussée postérieure est de préférence obtenue avec une force comprise entre 150 et 250 Newtons.

On réalise alors deux clichés, un cliché du membre lésé et un second cliché de l'autre membre présumé sain, puis on effectue une comparaison des deux clichés. Sur le cliché du genou de profil sous contrainte ainsi obtenue, on trace les axes et les repères qui permettront d'évaluer au millimètre près l'amplitude du tiroir antérieur.

A cet effet, on trace la ligne des plateaux superposés, puis on détermine le point situé à mi-chemin entre le rebord postérieur du plateau tibial interne et le rebord postérieur du plateau tibial externe. A partir de ce point, on abaisse une perpendiculaire à la ligne des plateaux, puis on identifie le point le plus postérieur de la ligne courbe, convexe vers l'arrière, que forment les rebords condyliens superposés. On évalue alors la distance de ce point à la perpendiculaire de la ligne des plateaux, ladite distance correspondant à la valeur au millimètre de l'amplitude du tiroir antérieur provoquée. La même mesure réalisée du côté sain donne le tiroir antérieur physiologique en valeur absolue. Ainsi, la soustraction du tiroir antérieur physiologique du tiroir antérieur pathologique donne le tiroir antérieur différentiel permettant à l'examinateur d'en déduire un diagnostic quant à une lésion du ligament croisé antérieur.

Ce type de dispositif et cette technique de radiographie dynamique mécanique de la lésion du LCA posent le problème du rayonnement X qui empêche des mesures itératives dans le cadre d'un suivi du patient, que ce soit en pré opératoire ou post-lésionnel, ou en post opératoire pour suivre l'évolution de la ligamentoplastie, par exemple.

Par ailleurs, ce type de dispositif présente l'inconvénient de fournir une faible sensibilité en raison des contractures réflexes des muscles jambiers lors de la poussée, qui ne permettent pas d'obtenir de bonnes mesures de la translation antérieure du tibia.

On connaît également un dispositif d'aide au diagnostic d'une lésion du ligament croisé antérieur, commercialisé sous la dénomination KT 1000 (marque déposée), qui est décrit dans le brevet américain US 4,583,555. La méthode consiste à fixer ledit appareil KT 1000 en le sanglant à l'aide de velcros® sur la jambe du patient à examiner et de tirer sur une poignée afin d'exercer une traction sur le tibia, la cuisse, c'est-à-dire le fémur, étant maintenue par un appui rotulien et le genou positionné à 20° de flexion grâce à un support réglable au-dessous et le pied étant en position neutre à l'aide d'une caisse située sur un autre support. Le dispositif comporte un support du membre inférieur comprenant un moyen de poussée au postéro supérieur du mollet et un capteur de déplacement du tibia venant se positionner sur la face antérosupérieure dudit tibia, sur la tubérosité tibiale antérieure. Ledit moyen de poussée engendre un déplacement du tibia et, par conséquent, du capteur qui permet de mesurer la translation du tibia par rapport au fémur.

Tous ces dispositifs de l'art antérieur présentent l'inconvénient de ne pas être réglables à distance, de ne pas pouvoir être utilisables en imagerie par résonance magnétique (IRM) et de ne pas permettre l'étude échographique des compartiments collatéraux et postérieurs du genou lors de leur utilisation.

US5662121A divulgue un dispositif constitué de deux cylindres et utilisant des forces hydrauliques pour avancer le tibia.

EP0568148A divulgue un dispositif de détection d'une lésion du genou comprenant un dispositif de déplacement du tibia. Pour effectuer une force sur le tibia, il emploie une poignée qui doit être tirée par l'expérimentateur. Un mécanisme similaire est aussi décrit dans US4583555A.

Le dispositif divulgué dans US4583554A emploie un levier situé au-dessous de la jambe pour relever le tibia.

DE3636843A1 divulgue un système de poulies et de poids pour effectuer un déplacement du tibia.

**BREVE DESCRIPTION DE L'INVENTION**

L'un des buts de l'invention est donc de remédier à tous ces inconvénients en proposant un dispositif de détection et du suivi d'une lésion du ligament croisé antérieur dit LCA du genou, de conception simple et peu onéreuse, d'utilisation simple, permettant un calcul aisé et rapide du tiroir antérieur ainsi que la possibilité d'utilisation du dispositif lors de l'acquisition d'images médicales par rayons X, par imagerie par résonance magnétique (IRM) et/ou par ultrasons afin de permettre l'étude échographique des compartiments collatéraux et postérieurs du genou.

A cet effet, et conformément à l'invention, il est proposé un dispositif pour effectuer une translation antérieure du tibia par rapport au fémur selon le test dit de Lachman, détection et de suivi d'une lésion, telle qu'une rupture, par exemple, du ligament croisé antérieur dit LCA du genou ; ledit dispositif est remarquable en ce qu'il est constitué d'au moins une pièce allongée concave, rigide, sensiblement hémicylindrique, dite coque, apte à coiffer le tibia d'un patient de telle manière que ses extrémités libres prennent appui respectivement sur le tibia en situation diaphysaire inférieure et sur la diaphyse du fémur et/ou sur la patella, en situation supérieure, la paroi concave de la coque formant un espace libre avec la partie haute du tibia, une poche gonflable apte à être positionnée sur la face convexe de la coque au niveau de la partie haute du tibia, connectée à une source de fluide sous pression, et à procurer une avancée tibiale qui reproduit le test de Lachman lors de l'expansion de ladite poche, et des moyens de fixation de la coque sur la jambe du patient.

Par ailleurs, la poche gonflable est solidarisée sur la face convexe de la coque, ladite poche gonflable étant de préférence collée sur la face convexe de la coque.

De manière particulièrement avantageuse, la poche gonflable est connectée par une tubulure souple à un manomètre.

Ledit manomètre consiste de préférence en un manomètre à commande manuelle.

De plus, les moyens de fixation consistent en au moins un brassard.

Selon une caractéristique essentielle du dispositif conforme à l'invention, la coque est obtenue dans un matériau radiotransparent et/ou amagnétique, et de préférence par thermoformage d'une résine polymère.

Une telle coque en résine polymère présente un encombrement très faible permettant son utilisation dans un dispositif d'acquisition d'images médicales tel qu'un dispositif d'IRM, et ce malgré l'étroitesse des antennes genou pour IRM.

Accessoirement, le bord périphérique de la coque est arrondi afin d'éviter toute lésion des tissus de la jambe lors de la mise en place du dispositif sur la jambe du patient.

Un autre objet de l'invention concerne un procédé pour effectuer une translation antérieure du tibia par rapport au fémur selon le test dit de Lachman, pour la détection et de suivi d'une lésion du genou, en particulier du ligament croisé antérieur dit LCA du genou. Ledit procédé est remarquable en ce qu'il comporte au moins une étape de positionnement d'une pièce allongée concave, rigide, sensiblement hémicylindrique, dite coque, sur le tibia d'un patient de telle manière que ses extrémités libres prennent appui respectivement sur le tibia en situation diaphysaire inférieure et sur la diaphyse du fémur et/ou sur la patella, en situation supérieure, la paroi concave de la coque formant un espace libre avec la partie haute du tibia, ladite coque comportant une poche gonflable positionnée sur la face convexe de la coque au niveau de la partie haute du tibia et connectée à une source de fluide sous pression, puis une étape de fixation de la coque sur la jambe du patient au moyen d'un brassard en entourant le mollet et en coiffant la poche gonflable, et finalement une étape d'alimentation en fluide sous pression de la poche gonflable procurant une expansion de ladite poche gonflable qui entraîne alors le brassard et donc le mollet vers l'avant, procurant ainsi une avancée tibiale qui reproduit le test de Lachman.

On notera que cette étape d'expansion de la poche gonflable peut avantageusement être obtenue progressivement permettant l'étude du ligament croisé antérieur lors de l'application d'une force croissante visant à déplacer progressivement le tibia en avant du fémur, ce déplacement correspondant au tiroir antéro-postérieur. Le procédé permet également d'obtenir une analyse vidéo de la mise en tension du ligament. A cet égard, lors de l'expansion progressive de la poche gonflable, des séquences IRM dites rapides sont acquises, puis les images des séquences IRM rapides sont assemblées pour obtenir une vidéo.

Un dernier objet de l'invention concerne un procédé de détermination du tiroir global et/ou du tiroir médial et/ou du tiroir latéral et/ou de la rotation fémoro-tibiale pour la détection et de suivi d'une lésion du genou, en particulier du ligament croisé antérieur dit LCA du genou ; ledit procédé est remarquable en ce qu'il comporte au moins une étape de positionnement d'une pièce allongée concave, rigide, sensiblement hémicylindrique, dite coque, sur le tibia d'un patient de telle manière que ses extrémités libres prennent appui respectivement sur le tibia en situation diaphysaire inférieure et sur la diaphyse du fémur et/ou sur la patella, en situation supérieure, la paroi concave de la coque formant un espace libre avec la partie haute du tibia, ladite coque comportant une poche gonflable positionnée sur la face convexe de la coque au niveau de la partie haute du tibia et connectée à une source de fluide sous pression, puis une étape de fixation de la coque sur la jambe du patient au moyen d'un brassard en entourant le mollet et en coiffant la poche gonflable, une étape d'acquisition d'images médicales en position de repos c'est-à-dire lorsque la poche gonflable n'est pas alimentée en fluide sous pression, puis une étape d'acquisition d'images médicales en position active c'est-à- dire lors de l'alimentation en fluide sous pression de la poche gonflable procurant une expansion de ladite poche gonflable qui entraîne alors le brassard et donc le mollet vers l'avant, procurant ainsi une avancée tibiale qui reproduit le test de Lachman, puis une étape de fusion des images acquises en position de repos et en position active, et finalement une étape de calcul du tiroir global et/ou du tiroir médial et/ou du tiroir latéral et/ou de la rotation fémoro-tibiale en mesurant sur les images fusionnées notamment le déplacement du tibia par rapport au fémur.

Les images acquises sont soit des images IRM soit des images modélisées en 3D à partir d'images radiographiques à rayons X ou d'images IRM.

**BREVE DESCRIPTION DES DESSINS**

D'autres avantages et caractéristiques ressortiront mieux de la description qui va suivre d'une variante d'exécution, donnée à titre d'exemple non limitatif, du dispositif conforme à l'invention, en référence aux dessins annexés, sur lesquels :

- la figure 1 est une vue en perspective éclatée du dispositif conforme à l'invention apte à être positionné sur la jambe d'un patient à examiner,
- la figure 2 est une vue en perspective de la coque du dispositif conforme à l'invention représentée sur la figure 1,
- la figure 3 est une vue de côté du dispositif conforme à l'invention positionné sur la jambe du patient lors d'un examen,
- la figure 4 est une vue de côté schématique de la coque munie de la poche gonflable positionnée sur la jambe du patient à examiner,
- la figure 5 est une représentation schématique, vue de côté, du dispositif conforme à l'invention lors de la mise sous pression de la poche gonflable.

**DESCRIPTION DETAILLEE DE L'INVENTION**

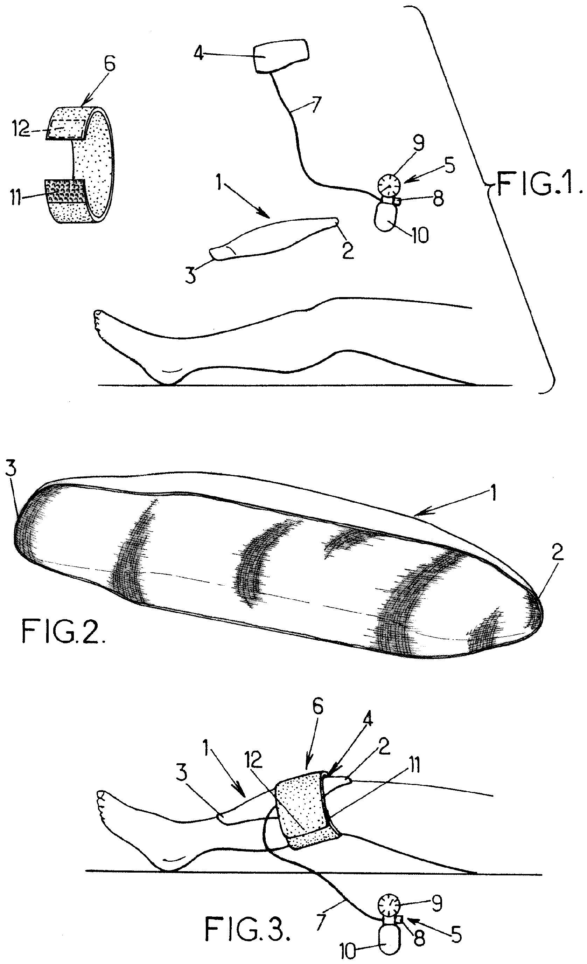

En référence aux figures 1 à 3, le dispositif conforme à l'invention comporte une pièce allongée concave, rigide, dite coque 1, apte à coiffer le tibia d'un patient de telle manière que ses extrémités libres proximale 2 et distale 3 prennent appui respectivement sur la patella, c'est-à-dire la rotule du patient, et sur le tibia en situation diaphysaire inférieure, une poche gonflable 4 apte à être positionnée sur la face convexe de la coque 1 au niveau de la partie haute du tibia, et connectée à une source de fluide sous pression 5, et des moyens de fixation 6 de la coque 1 sur la jambe du patient.

La coque 1 présente une forme allongée concave, rigide, sensiblement hémicylindrique, obtenue d'une seule pièce, qui vient s'appliquer à la face antérieure de la jambe. La coque 1 présente une faible épaisseur, de l'ordre de quelques millimètres, et est obtenue par moulage, et de préférence par thermoformage, à partir d'un modèle. Ladite coque 1 est de préférence obtenue dans un matériau amagnétique et radiotransparent, ledit matériau consistant par exemple dans une résine polymère. La forme de la coque 1 allongée concave, sensiblement hémicylindrique, et dont les extrémités libres proximal 2 et distal 3 sont légèrement aplaties, procure deux appuis, respectivement un appui proximal et un appui distal, fixes, libérant la face antérieure du tibia. Ainsi, lorsque la coque 1 est appliquée sur la face antérieure de la jambe du patient, ladite coque 1 entre en contact avec la patella en situation supérieure et avec le tibia en situation diaphysaire inférieure. Les extrémités proximales et distales 2 et respectivement 3, formant des appuis, épousent la forme des structures anatomiques en contact, offrant confort et stabilité. On observera que les courbes de la coque 1 permettent de dégager un espace libre 20 compris entre 15 et 40 mm, et de préférence un espace d'environ 20 mm, entre la paroi concave de la coque 1 et la partie haute du tibia du patient. Cet espace libre d'environ 20 mm permet le mouvement du tibia vers le haut lors de la manipulation en cas de rupture du ligament croisé antérieur comme on le verra plus loin.

On observera que l'extrémité distale 2 de la coque 1 pourra prendre appui sur la diaphyse du fémur et/ou sur la patella sans sortir du cadre de l'invention.

Par ailleurs, le bord périphérique de la coque 1 est avantageusement arrondi afin d'éviter toute lésion des tissus de la jambe lors de la mise en place de la coque 1 sur cette dernière.

De plus, de manière avantageuse, la paroi concave de la coque 1 s'étendant au contact du tibia du patient lorsque ladite coque est placée sur sa jambe est recouverte d'un tissu de préférence hypoallergénique afin de procurer un meilleur confort pour le patient lors de la manipulation.

Il va de soi que la totalité de la paroi concave de la coque 1 peut être recouverte d'un tissu sans sortir du cadre de l'invention.

En référence aux figures 1, 3 et 4, la poche gonflable 4 qui est obtenue dans un matériau souple, élastique, amagnétique et radiotransparent, tel qu'un matériau plastique, est solidarisée sur la face convexe de la coque 1 au niveau du tiers supérieur de cette dernière. Ainsi, la coque 1 ayant une longueur L, la poche gonflable 4 est positionnée à une distance d'environ $\dfrac{L}{3}$ depuis

l'extrémité proximale 2 de la coque 1 prenant appui sur la patella lorsque ladite coque 1 est positionnée sur la jambe du patient. Ladite poche gonflable 4 est de préférence collée sur la face convexe de la coque 1.

Toutefois, il est bien évident que la poche gonflable 4 pourra être solidarisée sur la face convexe de la coque 1 par tout moyen approprié tel que par exemple, par des moyens de fixation à boucles et crochets du type velcro (marque déposée) sans pour autant sortir du cadre de l'invention.

En référence aux figures 1 et 3, la poche gonflable 4 est connectée par une tubulure souple 7 à un manomètre manuel 8 comportant un cadran à aiguilles 9 indiquant la pression du fluide contenu dans la poche gonflable 4 et une poire 10 permettant de faire varier la pression de l'air dans ladite poche gonflable 4.

Il est bien évident que le manomètre manuel 8 pourra être substitué par toute autre source de fluide sous pression et tout autre moyen de mesure de la pression du fluide contenu dans la poche gonflable 4 sans pour autant sortir du cadre de l'invention. Par exemple, le cadran à aiguille 9 pourra être substitué par un cadran digital.

La tubulure 7 connectant le manomètre 8 à la poche gonflable 4 consiste en une tubulure en caoutchouc de longueur variable, le caoutchouc étant un matériau amagnétique et radiotransparent. On notera que ladite tubulure souple 7 présentera une longueur suffisante pour permettre un contrôle à la fois précis et à distance de la pression manométrique de la poche gonflable 4, et de préférence une longueur permettant de réaliser l'expansion de la poche gonflable 4 en dehors de la salle IRM depuis la console d'acquisition dudit dispositif d'acquisition.

Accessoirement, la tubulure souple 7 pourra comprendre plusieurs parties aptes à être assemblées les unes aux autres, de manière amovible par des moyens de connexion appropriés bien connus dans l'art antérieur.

En référence aux figures 1 et 3, la coque 1 est directement fixée à la jambe du patient grâce à un brassard 6 comportant sur sa face extérieure des moyens de fixation 11, telles que des boucles par exemple, aptes à coopérer avec des moyens de fixation complémentaires 12, tels que des crochets par exemple, positionnés sur la face intérieure dudit brassard 6 à proximité d'au moins l'une de ses extrémités libres, les moyens de fixation à boucles 11 et les moyens de fixation complémentaires 12 à crochets formant un ensemble de type velcro (marque déposée). Ledit brassard 6, en référence à la figure 3, est positionné de telle manière qu'il entoure le mollet de la jambe du patient en coiffant la poche gonflable 4 du dispositif.

On expliquera maintenant le fonctionnement du dispositif conforme à l'invention en référence aux figures 1 à 5.

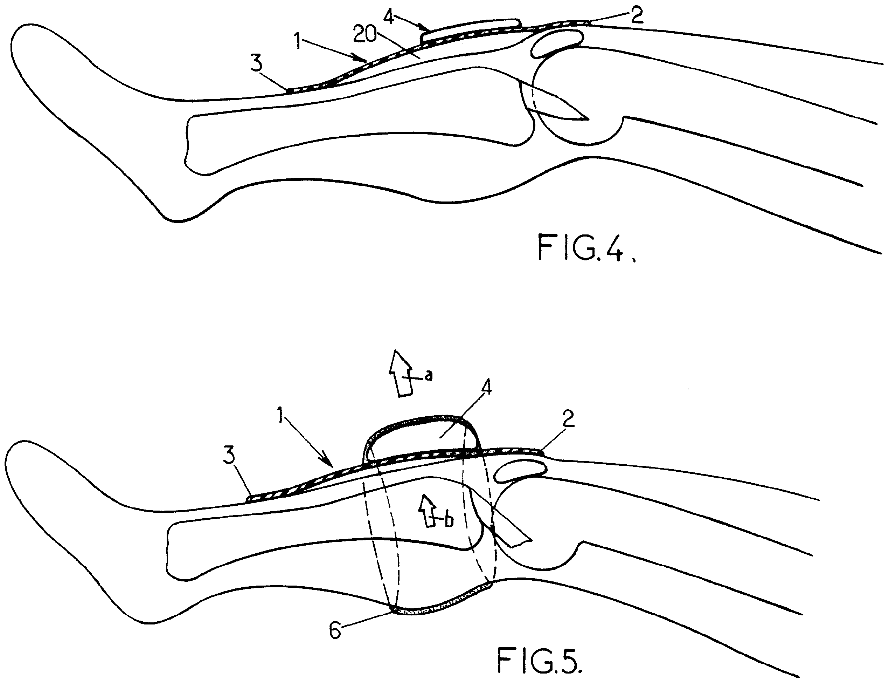

En référence à la figure 4, la coque 1 munie de la poche gonflable 4 est positionnée sur la jambe du patient à examiner de telle manière que l'extrémité proximale 2 prenne appui sur la patella et que l'extrémité distale 3 prenne appui sur le tibia en situation diaphysaire. La coque 1 est alors fixée sur la jambe du patient, en référence aux figures 2 et 4, au moyen du brassard 6 en entourant le mollet et en coiffant la poche gonflable 4 du dispositif. L'expérimentateur manipule alors la poire 10 du manomètre 8 pour procurer l'expansion de la poche gonflable 4 comme l'indique la flèche a de la figure 5. L'expansion de la poche gonflable 4 entraîne alors le brassard 6 et donc le mollet vers l'avant, comme l'indique la flèche b de la figure 5, procurant ainsi une avancée tibiale qui reproduit le test de Lachman.

On notera que les propriétés amagnétiques et radiotransparentes de la coque 1, de la poche gonflable 4 et de la tubulure 7 connectant la poche gonflable 4 au manomètre 8 permettent, simultanément à la reproduction du test de Lachman, l'acquisition d'images médicales par rayons X et/ou par imagerie par résonance magnétique (IRM) et/ou par ultrasons, la forme de la coque 1 permettant l'étude échographique des compartiments collatéraux et postérieurs du genou à examiner.

De plus, on observera que les images médicales pourront avantageusement être acquises au cours de l'expansion de la poche gonflable 4 sans nécessiter un repositionnement du patient entre deux acquisitions successives.

Enfin, il est bien évident que les exemples que l'on vient de donner ne sont que des illustrations particulières, en aucun cas limitatives quant aux domaines d'application de l'invention.

## Revendications

1. Dispositif pour effectuer une translation antérieure du tibia par rapport au fémur selon le test dit de Lachman, pour la détection et le suivi d'une lésion du genou, en particulier du ligament croisé antérieur dit LCA du genou, **caractérisé en ce qu'**il est constitué d'au moins :

   - une pièce allongée concave, rigide, sensiblement hémicylindrique, dite coque (1), apte à coiffer le tibia d'un patient de telle manière que ses extrémités libres (2,3) prennent appui respectivement sur le tibia en situation diaphysaire inférieure et sur la diaphyse du fémur et/ou sur la patella, en situation supérieure, la paroi concave de la coque (1) formant un espace libre (20) avec la partie haute du tibia,

- une poche gonflable (4) positionnée sur la face convexe de la coque (1) au niveau de la partie haute du tibia, connectée à une source de fluide sous pression (5) et à procurer une avancée tibiale qui reproduit le test de Lachman lors de l'expansion de ladite poche (4),

- des moyens de fixation (6) de la coque (1) sur la jambe du patient consistant en au moins un brassard (6) pour entourer le mollet et pour coiffer la poche gonflable (4).

2. Dispositif suivant la revendication 1, **caractérisé en ce que** la poche gonflable (4) est solidarisée sur la face convexe de la coque (1).

3. Dispositif suivant la revendication 2, **caractérisé en ce que** la poche gonflable (4) est collée sur la face convexe de la coque (1).

4. Dispositif suivant l'une quelconque des revendications 1 à 3, **caractérisé en ce que** la poche gonflable (4) est connectée par une tubulure souple (7) à un manomètre (8).

5. Dispositif suivant la revendication 4, **caractérisé en ce que** le manomètre (8) est un manomètre à commande manuelle.

6. Dispositif suivant l'une quelconque des revendications 1 à 5, **caractérisé en ce que** la coque (1) est obtenue dans un matériau radiotransparent.

7. Dispositif suivant l'une quelconque des revendications 1 à 6, **caractérisé en ce que** la coque (1) est obtenue dans un matériau amagnétique.

8. Dispositif suivant les revendications 6 et 7, **caractérisé en ce que** la coque (1) est obtenue par thermoformage d'une résine polymère.

9. Procédé pour effectuer une translation antérieure du tibia par rapport au fémur selon le test dit de Lachman, pour la détection et le suivi d'une lésion du genou, en particulier du ligament croisé antérieur dit LCA du genou, **caractérisé en ce qu'**il comporte au moins les étapes suivantes de:

    - positionnement d'une pièce allongée concave, rigide, sensiblement hémicylindrique, dite coque (1), sur le tibia d'un patient de telle manière que ses extrémités libres (2,3) prennent appui respectivement sur le tibia en situation diaphysaire inférieure et sur la diaphyse du fémur et/ou sur la patella, en situation supérieure, la paroi concave de la coque (1) formant un espace libre (20) avec la partie haute du tibia, ladite coque (1) comportant une poche gonflable (4) positionnée sur la face convexe de la coque (1) au niveau de la partie haute du tibia et connectée à une source de fluide sous pression (5),

- fixation de la coque (1) sur la jambe du patient au moyen d'un brassard (6) en entourant le mollet et en coiffant la poche gonflable (4),

- alimentation en fluide sous pression de la poche gonflable (4) procurant une expansion de ladite poche gonflable (4) qui entraîne alors le brassard (6) et donc le mollet vers l'avant, procurant ainsi une avancée tibiale qui reproduit le test de Lachman.

10. Procédé de détermination du tiroir global et/ou du tiroir médial et/ou du tiroir latéral et/ou de la rotation fémoro-tibiale pour la détection et le suivi d'une lésion du genou, en particulier du ligament croisé antérieur dit LCA du genou, **caractérisé en ce qu'**il comporte au moins les étapes suivantes de :

    - positionnement d'une pièce allongée concave, rigide, sensiblement hémicylindrique, dite coque, sur le tibia d'un patient de telle manière que ses extrémités libres prennent appui respectivement sur le tibia en situation diaphysaire inférieure et sur la diaphyse du fémur et/ou sur la patella, en situation supérieure, la paroi concave de la coque formant un espace libre avec la partie haute du tibia, ladite coque comportant une poche gonflable positionnée sur la face convexe de la coque au niveau de la partie haute du tibia et connectée à une source de fluide sous pression,

- fixation de la coque sur la jambe du patient au moyen d'un brassard en entourant le mollet et en coiffant la poche gonflable,

- acquisition d'images médicales en position de repos c'est-à-dire lorsque la poche gonflable n'est pas alimentée en fluide sous pression,

- acquisition d'images médicales en position active c'est-à-dire lors de l'alimentation en fluide sous pression de la poche gonflable procurant une expansion de ladite poche gonflable qui entraîne alors le brassard et donc le mollet vers l'avant, procurant ainsi une avancée tibiale qui reproduit le test de Lachman,

- fusion des images acquises en position de repos et en position active, et

- calcul du tiroir global et/ou du tiroir médial et/ou du tiroir latéral et/ou de la rotation fémoro-tibiale en mesurant sur les images fusionnées notamment le déplacement du tibia par rapport au fémur.

11. Procédé suivant la revendication 10 **caractérisé en**

**ce que** les images acquises sont des images IRM

12. Procédé suivant la revendication 10 **caractérisé en ce que** les images acquises sont des images modélisées en 3D à partir d'images radiographiques à rayons X ou d'images IRM.

## Patentansprüche

1. Vorrichtung zur Durchführung einer Verschiebung der Tibia nach vorn bezüglich des Femurs gemäß dem sogenannten Lachman-Test zum Nachweis und zur Überwachung einer Läsion des Knies, insbesondere des vorderen Kreuzbandes, VKB genannt, des Knies, **dadurch gekennzeichnet, dass** sie wenigstens aus Folgendem besteht:

    - einem konkaven, starren, im Wesentlichen halbzylinderförmigen lang gestreckten Teil, Schale (1) genannt, das geeignet ist, die Tibia eines Patienten derart zu bedecken, dass sich seine freien Enden (2, 3) auf der Tibia in unterer diaphysärer Lage und auf der Diaphyse des Femurs und/oder auf der Patella in oberer Lage abstützen, wobei die konkave Wand der Schale (1) mit dem hohen Teil der Tibia einen freien Raum (20) bildet,
    - einer aufblasbaren Tasche (4), die auf der konvexen Seite der Schale (1) im Bereich des hohen Teils der Tibia positioniert ist, mit einer Quelle von unter Druck stehendem Fluid (5) verbunden ist und dazu bestimmt ist, bei der Ausdehnung der Tasche (4) ein Vorschieben der Tibia zu bewirken, welches den Lachman-Test reproduziert,
    - Mittel zur Befestigung (6) der Schale (1) am Bein des Patienten, die aus wenigstens einer Manschette (6) zum Umschließen der Wade und zum Bedecken der aufblasbaren Tasche (4) bestehen.

2. Vorrichtung nach Anspruch 1, **dadurch gekennzeichnet, dass** die aufblasbare Tasche (4) mit der konvexen Seite der Schale (1) fest verbunden ist.

3. Vorrichtung nach Anspruch 2, **dadurch gekennzeichnet, dass** die aufblasbare Tasche (4) auf die konvexe Seite der Schale (1) geklebt ist.

4. Vorrichtung nach einem der Ansprüche 1 bis 3, **dadurch gekennzeichnet, dass** die aufblasbare Tasche (4) durch eine flexible Röhre (7) mit einem Manometer (8) verbunden ist.

5. Vorrichtung nach Anspruch 4, **dadurch gekennzeichnet, dass** das Manometer (8) ein manuell betätigtes Manometer ist.

6. Vorrichtung nach einem der Ansprüche 1 bis 5, **dadurch gekennzeichnet, dass** die Schale (1) aus einem strahlendurchlässigen Material hergestellt ist.

7. Vorrichtung nach einem der Ansprüche 1 bis 6, **dadurch gekennzeichnet, dass** die Schale (1) aus einem nichtmagnetischen Material hergestellt ist.

8. Vorrichtung nach den Ansprüchen 6 und 7, **dadurch gekennzeichnet, dass** die Schale (1) durch Warmformen eines Polymerharzes hergestellt ist.

9. Verfahren zur Durchführung einer Verschiebung der Tibia nach vorn bezüglich des Femurs gemäß dem sogenannten Lachman-Test zum Nachweis und zur Überwachung einer Läsion des Knies, insbesondere des vorderen Kreuzbandes, VKB genannt, des Knies, **dadurch gekennzeichnet, dass** es wenigstens die folgenden Schritte umfasst:

    - Positionierung eines konkaven, starren, im Wesentlichen halbzylinderförmigen lang gestreckten Teils, Schale (1) genannt, auf der Tibia eines Patienten, derart, dass sich seine freien Enden (2, 3) auf der Tibia in unterer diaphysärer Lage und auf der Diaphyse des Femurs und/oder auf der Patella in oberer Lage abstützen, wobei die konkave Wand der Schale (1) mit dem hohen Teil der Tibia einen freien Raum (20) bildet, wobei die Schale (1) eine aufblasbare Tasche (4) aufweist, die auf der konvexen Seite der Schale (1) im Bereich des hohen Teils der Tibia positioniert ist und mit einer Quelle von unter Druck stehendem Fluid (5) verbunden ist,
    - Befestigung der Schale (1) am Bein des Patienten mittels einer Manschette (6), wobei die Wade umschlossen wird und wobei die aufblasbare Tasche (4) bedeckt wird,
    - Zuführung von unter Druck stehendem Fluid zu der aufblasbaren Tasche (4), was eine Ausdehnung der aufblasbaren Tasche (4) bewirkt, welche dann die Manschette (6) und damit die Wade nach vorn mitnimmt, wodurch ein Vorschieben der Tibia bewirkt wird, welches den Lachman-Test reproduziert.

10. Verfahren zur Bestimmung der Gesamtschublade und/oder der medialen Schublade und/oder der lateralen Schublade und/oder der femorotibialen Rotation zum Nachweis und zur Überwachung einer Läsion des Knies, insbesondere des vorderen Kreuzbandes, VKB genannt, des Knies, **dadurch gekennzeichnet, dass** es wenigstens die folgenden Schritte umfasst:

    - Positionierung eines konkaven, starren, im Wesentlichen halbzylinderförmigen lang gestreckten Teils, Schale genannt, auf der Tibia eines Patienten, derart, dass sich seine freien Enden auf der Tibia in unterer diaphysärer Lage und auf der Diaphyse des Femurs und/oder auf der Patella in oberer Lage abstützen, wobei die konkave Wand der Schale mit dem hohen Teil der Tibia einen freien Raum bildet, wobei die Schale eine aufblasbare Tasche aufweist, die auf der konvexen Seite der Schale im Bereich des hohen Teils der Tibia positioniert ist und mit einer Quelle von unter Druck stehendem Fluid verbunden ist,

- Befestigung der Schale am Bein des Patienten mittels einer Manschette, wobei die Wade umschlossen wird und wobei die aufblasbare Tasche bedeckt wird,

- Erfassung von medizinischen Bildern in der Ruheposition, das heißt wenn der aufblasbaren Tasche kein unter Druck stehendes Fluid zugeführt wird,

- Erfassung von medizinischen Bildern in der aktiven Position, das heißt während der Zuführung von unter Druck stehendem Fluid zu der aufblasbaren Tasche, was eine Ausdehnung der aufblasbaren Tasche bewirkt, welche dann die Manschette und damit die Wade nach vorn mitnimmt, wodurch ein Vorschieben der Tibia bewirkt wird, welches den Lachman-Test reproduziert,

- Überlagerung der in der Ruheposition und in der aktiven Position erfassten Bilder, und

- Berechnung der Gesamtschublade und/oder der medialen Schublade und/oder der lateralen Schublade und/oder der femorotibialen Rotation durch Messen insbesondere der Verlagerung der Tibia bezüglich des Femurs auf den überlagerten Bildern.

11. Verfahren nach Anspruch 10, **dadurch gekennzeichnet, dass** die erfassten Bilder MRT-Bilder sind.

12. Verfahren nach Anspruch 10, **dadurch gekennzeichnet, dass** die erfassten Bilder Bilder sind, die ausgehend von Röntgenbildern oder MRT-Bildern in 3D modelliert wurden.

## Claims

1. Device for performing an anterior translation of the tibia with respect to the femur according to the so-called Lachman test, for the detection and monitoring of a knee injury, in particular of the anterior cruciate ligament, referred to as the ACL, of the knee, **characterized in that** it is constituted by at least:

   - an elongated, concave, rigid, substantially semicylindrical part, referred to as a shell (1), suitable for covering the tibia of a patient in such a way that its free ends (2, 3) rest respectively on the tibia in a lower diaphyseal position and on the diaphysis of the femur and/or on the patella, in an upper position, the concave wall of the shell (1) forming a free space (20) with the upper part of the tibia,

   - an inflatable pouch (4) positioned on the convex face of the shell (1) at the level of the upper part of the tibia, connected to a source of pressurized fluid (5), for producing a forward movement of the tibia that reproduces the Lachman test during the expansion of said pouch (4),

   - means (6) for fastening the shell (1) onto the leg of the patient, consisting of at least one cuff (6) for surrounding the calf and for covering the inflatable pouch (4).

2. Device according to claim 1, **characterized in that** the inflatable pouch (4) is firmly fixed to the convex face of the shell (1).

3. Device according to claim 2, **characterized in that** the inflatable pouch (4) is bonded onto the convex face of the shell (1).

4. Device according to any one of claims 1 to 3, **characterized in that** the inflatable pouch (4) is connected by flexible tubing (7) to a manometer (8).

5. Device according to claim 4, **characterized in that** the manometer (8) is a manometer with manual control.

6. Device according to any one of claims 1 to 5, **characterized in that** the shell (1) is obtained in a radiotransparent material.

7. Device according to any one of claims 1 to 6, **characterized in that** the shell (1) is obtained in a non-magnetic material.

8. Device according to claims 6 and 7, **characterized in that** the shell (1) is obtained by thermoforming of a polymer resin.

9. Method for carrying out an anterior translation of the tibia with respect to the femur according to the so-called Lachman test,
for the detection and monitoring of a knee injury, in particular of the anterior cruciate ligament, referred to as the ACL, of the knee, **characterized in that** it includes at least the following steps of:

   - positioning an elongated, concave, rigid, substantially semicylindrical part referred to as a shell (1), on the tibia of a patient in such a way that its free ends (2, 3) rest respectively on the tibia in a lower diaphyseal position and on the diaphysis of the femur and/or on the patella, in an upper position, the concave wall of the shell (1) forming a free space (20) with the upper part of the tibia, said shell (1) comprising an inflatable pouch (4) positioned on the convex face of the shell (1) at the level of the upper part of the tibia and connected to a source of pressurized fluid (5),

- fastening the shell (1) onto the leg of the patient by means of a cuff (6) surrounding the calf and covering the inflatable pouch (4),

- supplying the inflatable pouch (4) with pressurized fluid, producing expansion of said inflatable pouch (4) which then drives the cuff(6), and therefore the calf, forwards, thus producing a forward movement of the tibia which reproduces the Lachman test.

10. Method for determining the overall drawer and/or the medial drawer and/or the lateral drawer and/or the femoro-tibial rotation for the detection and monitoring of a knee injury, in particular of the anterior cruciate ligament, referred to as the ACL, of the knee **characterized in that** it comprises at least the following steps of:

- positioning an elongated, concave, rigid, substantially semicylindrical part, referred to as a shell, on the tibia of a patient in such a way that its free ends rest respectively on the tibia in a lower diaphyseal position and on the diaphysis of the femur and/or on the patella, in an upper position, the concave wall of the shell forming a free space with the upper part of the tibia, said shell comprising a inflatable pouch positioned on the convex face of the shell at the level of the upper part of the tibia and connected to a source of pressurized fluid,

- fastening the shell onto the leg of the patient by means of a cuff surrounding the calf and covering the inflatable pouch,

- capturing medical images in the rest position i.e. when the inflatable pouch is not supplied with pressurized fluid,

- capturing medical images in the active position i.e. during the supply of pressurized fluid to the inflatable pouch, producing expansion of said inflatable pouch which then drives the cuff and therefore the calf forwards, thus producing a forward movement of the tibia which reproduces the Lachman test,

- merging the images captured in the rest position and in the active position, and

- calculation of the overall drawer and/or the medial drawer and/or the lateral drawer and/or the femoro-tibial rotation by measuring on the merged images in particular the displacement of the tibia with respect to the femur.

11. Method according to claim 10 **characterized in that** the captured images are MRI images.

12. Method according to claim 10 **characterized in that** the captured images are images modelled in 3D from X-ray radiographic images or MRI images.

FIG.1.

FIG.2.

FIG.3.

FIG.4.

FIG.5.

EP 2 408 366 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4583555 A
- US 5662121 A
- EP 0568148 A
- US 4583554 A
- DE 3636843 A1